(19) Europäisches Patentamt
European Patent Office
Office européen des brevets

(11) **EP 4 265 268 A2**

(12) **EUROPEAN PATENT APPLICATION**
published in accordance with Art. 153(4) EPC

(43) Date of publication:
**25.10.2023  Bulletin 2023/43**

(21) Application number: **21865362.4**

(22) Date of filing: **23.11.2021**

(51) International Patent Classification (IPC):
**A61K 38/16** *(2006.01)*     **A61P 35/00** *(2006.01)*

(52) Cooperative Patent Classification (CPC):
**A61P 35/00; A61K 38/16; A61K 38/162**

(86) International application number:
**PCT/CU2021/050013**

(87) International publication number:
**WO 2022/127945 (23.06.2022 Gazette 2022/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.12.2020  CU 20200103**

(71) Applicant: **Centro de Ingeniería Genética y
Biotecnología
La Habana 11600 (CU)**

(72) Inventors:
  • **AGUILAR NORIEGA, Daylen**
    **33400 Mayabeque (CU)**
  • **PEREA RODRÍGUEZ, Silvio, Ernesto**
    **11600 La Habana (CU)**

  • **PERERA NEGRIN, Yasser**
    **11600 La Habana (CU)**
  • **VÁZQUEZ BLOMQUIST, Dania, Marcia**
    **11300 La Habana (CU)**
  • **LEMOS PÉREZ, Gilda**
    **11600 La Habana (CU)**
  • **BALADRON CASTRILLO, Idania, Caridad**
    **10400 La Habana (CU)**
  • **DÍAZ REYES, Pablo, Arsenio**
    **10400 La Habana (CU)**

(74) Representative: **V.O.
P.O. Box 87930
2508 DH Den Haag (NL)**

Remarks:
The complete document including Reference
Table(s) and the Sequence Listing(s) can be
downloaded from the EPO website

(54) **CIGB-300 FOR USE IN THE THE INDUCTION OF ANTITUMOR AND ANTIVIRAL IMMUNTY**

(57)    Use of the peptide identified as SEQ ID NO: 1 for manufacturing a medicine which induces antitumor and antiviral immunity by immunogenic cell death. The invention also provides a method for treating cancer or viral infections by using therapeutically effective amounts of a drug that comprises the synthetic peptide of the invention. This method induces antitumor and antiviral immunity by immunogenic cell death. Also, the invention comprises the pharmaceutical combination of the peptide identified as SEQ ID NO: 1 and cancer immunotherapy approaches, which achieve stronger immunotherapeutic management to combat this disease.

EP 4 265 268 A2

## Description

### Technical field

The present invention is related to cancer immunotherapy and antiviral therapy field. In particular, it is based on the use of the peptide identified as SEQ ID NO: 1 for inducing antitumor and antiviral immunity by increasing immunogenic cell death. Also, it relates to the combination of the peptide with cancer immunotherapy.

### Prior Art

Cancer is one of the leading causes of death worldwide. The global incidence of cancer continues to grow, with new cases estimated to exceed 21.7 million by 2030 and there will be 13 million deaths from cancer per year (Tartari F and cols. Cancer Treat Rev 2016; 48:20-24).

One of the strategies in the development of new cancer treatment in the last 20 years has been the development of drugs whose mechanism is target-specific to interfere with the biochemical events involved with the viability of the tumor cell, thus producing apoptosis in the same. Generally, this class of drug is employed in combination with standard chemotherapy in first or second line of treatment of cancer patients, both in advanced and early stages.

The synthetic peptide identified as SEQ ID NO: 1 is a therapeutic candidate for cancer treatment, its primary action mechanism is to inhibit protein kinase CK2-mediated phosphorylation, thus leading to tumor cell death by apoptosis. It has shown antitumor effect in experimental oncology animal models (Perea SE and cols. Cancer Res 2004; 64:7129-9). Recently, direct antiviral effect of the peptide identified as SEQ ID NO: 1 has also been observed in different *in vitro* viral models. From a mechanistic point of view, it has been found that the peptide identified as SEQ ID NO: 1 interacts with B23/Nucleophosmin protein in the tumor cell nucleolus, inhibits its phosphorylation and induces nucleolar disassembly as apoptosis prelude (Perera Y and cols. Mol Cancer Ther 2009; 8(5)). Consistent with such molecular events, the peptide identified as SEQ ID NO: 1 modulates a series of proteins linked to different cellular processes and inhibits lung metastases colonization and tumor angiogenesis in preclinical cancer models (Farina HG and cols. Exp Cell Res 2011; 317:1677-1688) (Benavent Acero F and cols. Lung Cancer 2017; 107:14-21). Furthermore, the peptide identified as SEQ ID NO: 1 inhibits phosphorylation of other CK2 substrates such as AKT and PTEN in chronic lymphocytic leukemia cells (Martins LR and cols. Oncotarget 2014; 5:258-263). In clinical field, exploration of safety and tolerability of the peptide identified as SEQ ID NO: 1 in cancer patients has begun, where a trend of increased survival has been observed in patients with advanced disease, and above life expectancy (Batista-Albuerne N and cols. J Med Oncol 2018; 1:4). These studies have made it possible to know pharmacokinetics and biodistribution of the peptide identified as SEQ ID NO: 1, after its local or intravenous administration, as well as safe dose ranges and toxicity linked to research drug (Solares AM and cols. BMC Cancer 2019; 9(1):146) (Sarduy MR and cols. Br J Cancer 2015; 112:1636-43).

The highest aspiration of clinical immunologists and oncologist for many years has been trying to involve the immune system of the body in the neoplastic disease control. In line with this thinking, immunotherapy is presented as a promising option that is revolutionizing cancer treatment through discovery and development of new approaches that activate and strengthen antitumor immunity in cancer patients (Zugazagoitia J and cols. Clin Ther 2016; 38(7):1551-1566) (Yang Y. J Clin Invest 2015; 125(9):3335-3337).

At present, main immunotherapy options for cancer treatment are: monoclonal antibodies, immune checkpoint inhibitors, cancer vaccines and cell-based immunotherapy (Kenderian SS and cols. Biol Blood Transplant 2017; 23:235-246) (Ruella M and Kenderian SS. BioDrug 2017; 31(6):473-481). In addition, there are other non-specific immunotherapies, which activate the immune system in a general way, and this could thus attack tumor cells (Klener P Jr and cols. Curr Pharm Biotechnol 2015; 16(9):771-781) (Lee VC. P&T 2017; 42(6):375-383). Although chemotherapeutic drugs and radiotherapy do not have a direct stimulatory effect on cellular immunity, today it is known that some of these approaches can increase it indirectly, by inducing immunogenic cell death in the tumor cell. In the last years, the number of drugs that induce immunogenic cell death has been increased. Those include: anthracyclines (doxorubicin (DOX), epirubicin, idarubicin), oxaliplatin, cyclophosphamide, bortezomib, mitoxantrone and bleomycin (Garg AD and cols. Oncoimmunology 2017; 6(12): e1386829). Additionally, physical therapeutic methods such as radiotherapy, hypericin-based photodynamic therapy and high hydrostatic pressures, as well as some oncolytic viruses and microtubular inhibitors have been also shown to induce this type of cell death (Li X. Tumori Jornal 2018; 104(1):1-8).

In the past, necrosis was the only type of cell death considered immunogenic, producing unwanted inflammatory reactions, due to rapid release of various intracellular factors, cytokines and other inflammatory mediators (Rock KL and Kono H. Annu Rev Pathol 2008; 3:99-126). Conversely, apoptosis was considered a physiological process of cell death that is mostly tolerogenic or silent from immunological point of view (Matzinger P. Science 2002; 296:301-305). During late-phase apoptosis, cells are rapidly and specifically recognized by macrophages and other phagocytic cells, due to expression of various "eat me" signals such as calreticulin (CRT), Erp57 and HSP90 on the membrane (Hou W and cols. Cell Death and Disease 2013; 4, e966) and concomitant suppression of "don't eat me" signals, such as CD47 expression (Liu X and cols. Jornal of Hematology & Oncology 2017; 10:12). The latter is a molecule that is expressed on tumor cells and has been considered as an immune checkpoint for tumor evasion, as it constitutes an antiphagocytic signal for macrophages and dendritic cells (Tong B and Wang M. Future Oncol. 2018; 14(21):2179-2188) (WeiskopfK and cols. Science 2013; 341(6141):88-91).

Compared to classic tolerogenic apoptosis, immunogenic cell death activates the host's immune system and increases the immune response to immunotherapy, such as the response to dendritic cells-based cancer vaccines (Vandenberk L and cols. Front Immunol 2016; 6:663). In fact, tumor cells in process of immunogenic cell death, induced by drugs *in vitro,* are capable to induce an anticancer vaccine effect when they are implanted subcutaneously in immunocompetent mice (Keep O and cols. Oncoimmunology. 2014, 3:9). In this case, dendritic cells play a central role in the recognition of apoptotic cells, and in the initiation of an effective antitumor immune response (Ma Y and cols. Immunity 2013; 38:729-41). One of fundamental characteristics of damaged and dying cells is the exposition on membrane or secretion of molecules normally hidden in living and healthy cells, which acquire immunostimulatory properties. These molecules belong to well-known damage associated molecular pattern and could exert several effects on antigen presenting cells, including maturation, activation and antigen processing/presentation (Zitvogel L and cols. Cell. 2010; 140: 798-804). However, antitumor immunity associated with immunogenic cell death is rarely achieved in cancer patients, due to inability of anticancer drugs to produce this type of death at concentration that are lower than maximum tolerated dose *in vivo* (Montico B and cols. Int J Mol Sci. 2018; 19:594-609). This obstacle has been overcome with *ex vivo* treatment of tumor cell from patient, with high doses of drug that induce of immunogenic apoptosis, for generating autologous dendritic cells for vaccination purposes (Chen HM and cols. Cancer Immunol Immunother. 2012; 61:1989-2002) (Montico B and cols. Oncoimmunology. 2017; 6:e1356964). However, surgical resection of patient tumor is not always possible to achieve *ex vivo* procedure that allows obtaining autologous dendritic cells, especially in advanced stages of disease, or due to tumor anatomical location.

Therefore, at present, a major limitation of using immunogenic apoptosis inducers as adjuvant to dendritic cells-mediated vaccines consists of impossibility of using such drugs *in vivo* without the previous *ex vivo* activation procedure.

**Description of the invention** The present invention solves the aforementioned problem by providing the use of the peptide identified as SEQ ID NO: 1 to manufacture a medicine for inducing antitumor and antiviral immunity by increasing immunogenic cell death. The peptide identified as SEQ ID NO: 1 is a proapoptotic peptide, comprising the synthetic peptide initially called P15 fused to the cell-penetrating peptide Tat (amino acids 48-60), to facilitate internalization into cells (Perea SE and cols. Cáncer res. 2004; 64: 7127-7129). Unexpectedly, it is shown that this peptide is able to induce antitumor immunity activation, by activating "eat me" signals and reducing "don't eat me" signals. In particular, the invention demonstrates that there is activation of antitumor adaptive immune response, where there is maturation and activation of dendritic cells and cellular immune response mediated by CD8+ cytotoxic T lymphocytes. The effect described for the peptide identified as SEQ ID NO: 1 in the present invention is not observed for other inhibitors of CK2-mediated phosphorylation, such as the chemical compound CX4945, which directly block catalytic subunit of CK2 protein kinase. This effect is neither observed when cell-penetrating peptide Tat is employed, which has been evaluated as a negative control.

In one embodiment of the invention, the peptide identified as SEQ ID NO: 1 is used for the manufacture of a drug for the induction of antitumor and antiviral immunity by *in vivo* and *in vitro* activation and differentiation of dendritic cells. *In vitro* treatment is understood to mean the incubation in culture of tumor cells with the peptide identified as SEQ ID NO: 1. *Ex vivo* treatment refers to *in vitro* co-incubation of tumor cells from patients treated with the peptide identified as SEQ ID NO: 1 in the laboratory, together with dendritic cells from de same patients. In relation to *in vivo* treatment, an increase in "eat me" signals and a decrease in "don't eat me" signals are observed in the tumor, with subsequent activation of cellular immune response, both at systemic and intratumoral level. In the invention, it is shown for first time that the peptide identified as SEQ ID NO: 1 is capable of inducing immunogenic cell death of tumor cells, which administered in a vaccination schedule in mice induces *in vivo* immune response that protects against further challenge with live tumor cells, impairing tumor debut and growth.

In an embodiment of the invention, the induction of antitumor immunity by the use of the peptide identified as SEQ ID NO: 1 comprises the enhancement of the NK or cytotoxic T cell activity at tumor site. The use of the peptide identified as SEQ ID NO: 1 allows increasing the activity of cytotoxic T cells through adequate maturation and activation of dendritic cells. The peptide identified as SEQ ID NO: 1 is capable of increasing the secretion of HMGB-1 and adenosine triphosphate (ATP) by tumor cells after treatment, which act as chemoattractant for antigen presenting cells, favoring their recruitment, maturation and activation at the tumor site. This make the peptide identified as SEQ ID NO: 1 a suitable compound to enhance the effect of vaccines based on dendritic cells or to contribute to *in situ* vaccination with these cells.

Contrary to other antitumor agents that induce immunogenic cell death, which only achieve activation of dendritic cells when they are incubated *ex vivo* with tumor cells, the use of the peptide identified as SEQ ID NO: 1 solves said limitation by inducing *in vivo* activation of dendritic cells, without need to isolate cells from the patients to do the procedure. This effect is observed when the peptide identified as SEQ ID NO: 1 is administered both intratumorally and systemically, which makes it an effective treatment for both leukemias and solid tumors, including those hard to access.

In a materialization of the invention, the induction of antitumor immunity by the used of the peptide identified as SEQ ID NO: 1 comprises the reversion of immunosuppressive to immunostimulatory tumor microenvironment. This synthetic peptide enhances the recruitment of immune cells capable of orchestrating a specific antitumor response and decreasing the present of regulatory T cells.

In a realization of the invention, the peptide achieves the induction of antitumor and antiviral immunity by increasing pro-inflammatory cytokines such as TNF-α, IL-6 and IL-12, capable of increasing the expression of MHC-I on antigen-presenting cells, and promote T cells differentiation and NK cells activation. The increase in pro-inflammatory cytokines is accompanied by the decrease in antiinflammatory cytokines, such as IL-10 which limits the immune response generated and contributes to tumor progression or viral infection.

In a particular embodiment, the induction of antiviral immunity comprises the enhancement of specific humoral immune response against viral antigens, as demonstrated for the first time in the present invention. The ability of the peptide identified as SEQ ID NO: 1 to enhance the immune response makes it an ideal candidate to treat infectious diseases, particularly during viral infections. The invention also provides a method for treating cancer or viral infection by using therapeutically effective amounts of a drug that induces antitumor and antiviral immunity by immunogenic cell death where the drug comprises the synthetic peptide of the invention SEQ ID NO: 1.

The method of the present invention permits to decrease "don't eat me" signals like CD47 expression, which, together with the increase in "eat me" signals, favor phagocytosis of tumor cells by dendritic cells and M1 macrophages. This M1 macrophage function is relevant to their antiangiogenic activity that finally inhibits tumor progression. Therefore, the effect of the peptide of the invention eliminates the need to use additional CD47 inhibitors for enhancing the antitumor effect of certain drugs and reduces the number of compounds required to achieve an effective antitumor response. Thus, in one embodiment of the method of the invention, the induction of antitumor and antiviral immunity comprises the activation and differentiation of dendritic cells *in vivo* and *in vitro*. In another aspect, the use of the peptide identified as SEQ ID NO: 1 increase "eat me" signals such as CRT. This increases the ability of antigen-presenting cells to capture apoptotic tumor cells. The increase in "eat me" signals induced by the peptide identified as SEQ ID NO: 1 also increases the susceptibility of tumor cells to NK cytotoxicity, which favors the diversification of peptide-induced response and increases the antitumor efficiency. Therefore, in a materialization of the method of the invention, the induction of antitumor immunity comprises the enhancement of NK and cytotoxic T cells activity at tumor site, as well as the reversion of immunosuppressive to immunostimulatory tumor microenvironment, and increased secretion of pro-inflammatory cytokines. In another embodiment of the method of the invention, the induction of antiviral immunity comprises the enhancement of a specific humoral immune response against viral antigens.

In one embodiment of the invention, the drug comprising the peptide identified as SEQ ID NO: 1 enhances the effect of a vaccine used in cancer immunotherapy. The drug comprising the peptide identified as SEQ ID NO: 1 and the vaccine used in cancer immunotherapy can be administered sequentially or simultaneously in the course of the same treatment. In a particular embodiment, the vaccine used in cancer immunotherapy is a vaccine based on dendritic cells, cytotoxic T cells or tumor-infiltrating lymphocytes (TILs).

In the present invention, the use of the peptide identified as SEQ ID NO: 1 as an adjuvant to enhance the cellular immune response of antitumor vaccines is described for the first time. To do that, the peptide can be administered prior to vaccination by intratumoral or systemic route, which allows it to be used for both leukemias and solid tumor. It is necessary to highlight that the adjuvant effect was only observed for the peptide identified as SEQ ID NO: 1 and not for another inhibitor of phosphorylation mediated by CK2, like CX4945.

Another object of the present invention is a pharmaceutical combination that comprises the peptide identified as SEQ ID NO: 1 and a vaccine for cancer immunotherapy. In a materialization of the invention, the vaccine used in cancer immunotherapy is a vaccine based on dendritic cells, cytotoxic T cells or TILs. The ability to increase tumor infiltration of cytotoxic T lymphocytes, and decrease the presence of regulatory cells, makes the peptide identified as SEQ ID NO: 1 a suitable compound enhancing the effect of vaccines based on cytotoxic T lymphocytes (CTL) and TILs, thus leading to a more effective immunotherapeutic approach to treat cancer.

In one embodiment of the invention, the drug comprising the peptide identified as SEQ ID NO: 1 enhances the effect of a PDL1 immune checkpoint inhibitor used in cancer immunotherapy. In a materialization of the invention, the peptide identified as SEQ ID NO: 1 and the PDL1 immune checkpoint inhibitor are administered sequentially or simultaneously in the course of the same treatment. In a particular embodiment, the PDL1 immune checkpoint inhibitor is an anti-PDL1 monoclonal antibody.

In another aspect, the invention discloses a pharmaceutical combination comprising the peptide identified as SEQ ID NO: 1 and a PDL1 immune checkpoint inhibitor used in cancer immunotherapy. In an embodiment of the invention, in the pharmaceutical combination, the PDL1 immune checkpoint inhibitor is an anti-PDL1 monoclonal antibody.

For instance, in patients with non-small cell lung cancer lacking actionable mutations, the most effective therapy is PDL1 immune checkpoint inhibitors. However, its efficacy depends on the level of expression of this molecule in the tumor. The use of the peptide identified as SEQ ID NO: 1 in combination with a PDL1 inhibitor solves the problem by increasing PDL1 expression and therefore increasing the clinical efficacy of the PDL1 inhibitor. This combination offers a novel therapeutic strategy for cancer treatment.

**Brief description of the figures**

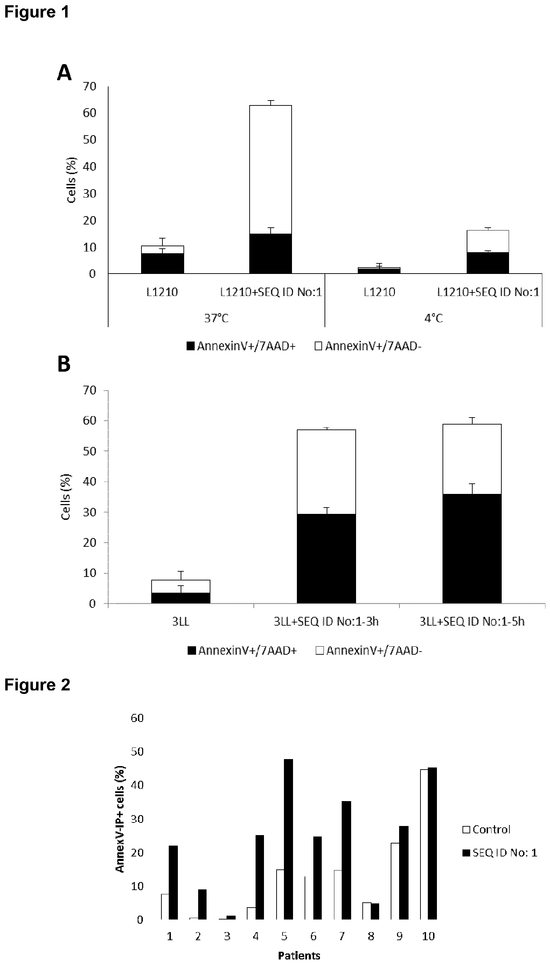

**Figure 1.** Evaluation of induction of cell death in L1210 (**A**) and 3LL (**B**) cell lines by the peptide identified as SEQ ID NO: 1. Bar graphs indicate frequency of dead cells (Annexin V+/7AAD+) and dying cells (Annexin V/7AAD-) after treatment with the peptide identified as SEQ ID NO: 1. The error bars in positive direction indicate standard deviations from mean of replicates.

**Figure 2.** Evaluation of *ex vivo* apoptosis induction by the peptide identified as SEQ ID NO: 1 in cells from patients with malignant blood diseases. The figure shows percentage of Annexin V/PI+ cells from ten patients with malignant blood diseases after treatment *ex vivo* with the peptide identified as SEQ ID NO: 1 for 48 hours.

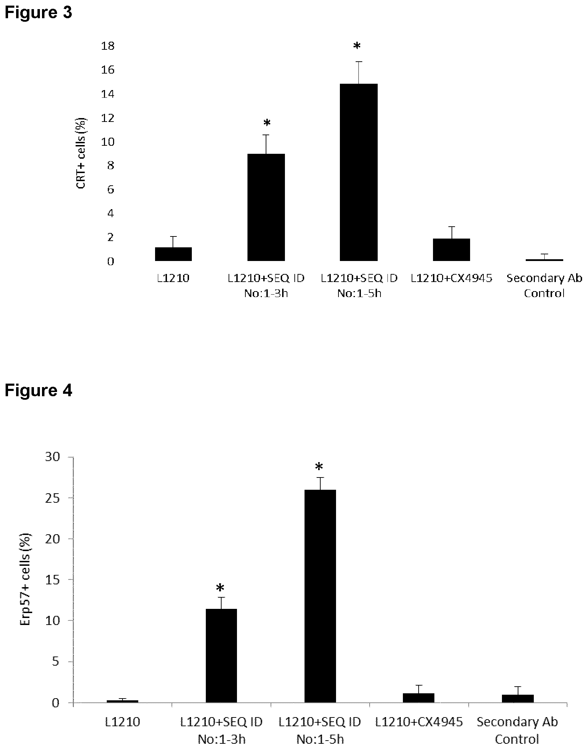

**Figure 3.** Flow cytometry analysis of CRT translocation on cellular membrane in L1210 cells treated with the peptide identified as SEQ ID NO: 1. The figure shows percentage of CRT+ cells upon treatment with the peptide identified as SEQ ID NO: 1 and CX4945. The graph is represented as mean $\pm$ standard deviation of six replicates of three independent experiments. The asterisk indicates statistically significant differences in percentage of CRT+ cells respect to cells without treatment and those treated with CX4945 (Fisher's exact test, *p<0.05*).

**Figure 4.** Flow cytometry analysis of Erp57 translocation on membrane of L1210 cells upon treatment with the peptide identified as SEQ ID NO: 1. The figure shows percentage of Erp57+ cells upon treatment with the peptide identified as SEQ ID NO: 1 and CX4945. Graph is represented as mean $\pm$ standard deviation of six replicates of three independent experiments. The asterisk indicates statistically significant differences regarding percentage of Erp57+ cells with respect to cells without treatment and those treated with CX4945 (Fisher's exact test, *p<0.05*).

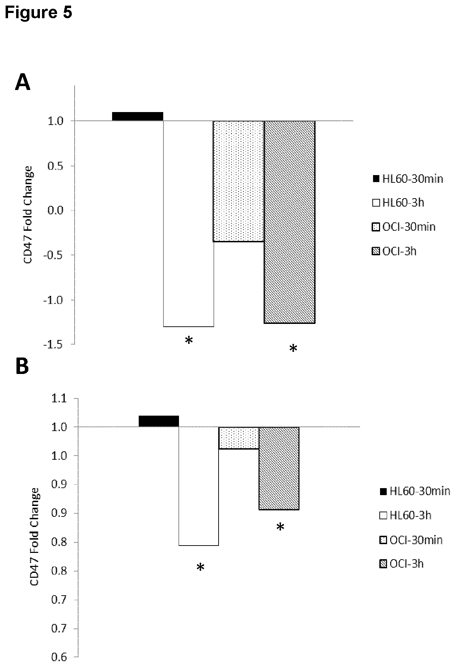

**Figure 5.** CD47 expression on HL60 and OCI-AML3 cells upon treatment with the peptide identified as SEQ ID NO: 1. Microarray analysis (**A**) and quantitative PCR (qPCR) (**B**). Bars represent change fold of CD47 gene expression relative to untreated cells, results were normalized with three reference genes *GAPDH, DDX5* y *ABL1*. Data was processed using REST 2009 v2.0.13 program. The asterisk indicates statistically significant.

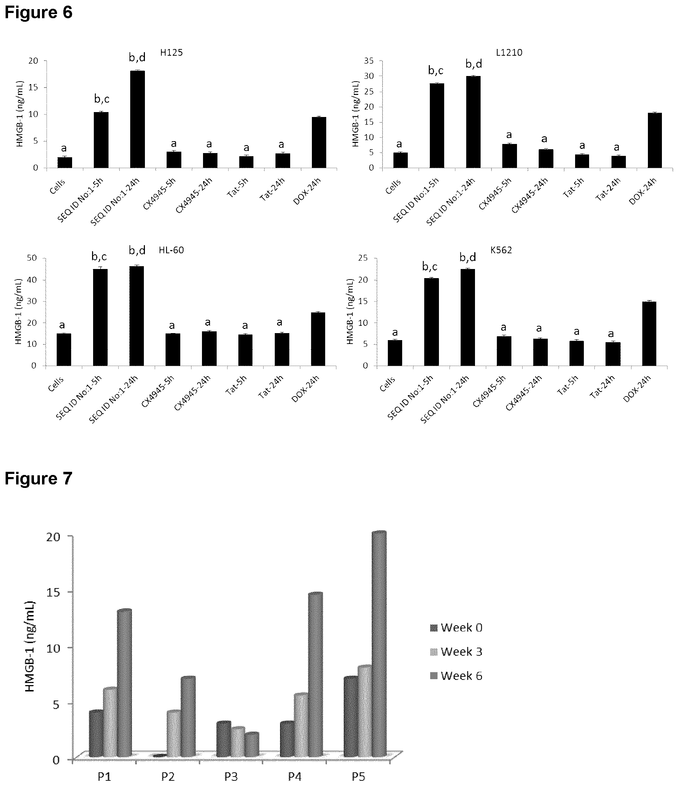

**Figure 6.** Evaluation of HMGB-1 secretion in culture supernatant of several tumor cell lines treated with the peptide identified as SEQ ID NO: 1. The graphs represent HMGB-1 levels determined by ELISA from culture supernatant of different tumor cell lines. Bar graphs represent mean $\pm$ standard deviation of three independent replicates. Statistical significance was determined using Kruskal-Wallis test and Dunn's multiple comparisons. Different letters indicate statistically significant differences: *p<0.05.*

**Figure 7.** Detection of HMGB-1 in serum from patients with acute myeloid leukemia treated with the peptide identified as SEQ ID NO: 1. The graph represents HMGB-1 levels determined by ELISA from serum of five patients treated with the peptide. Samples were taken at weeks 0, 3 and 6 after treatment.

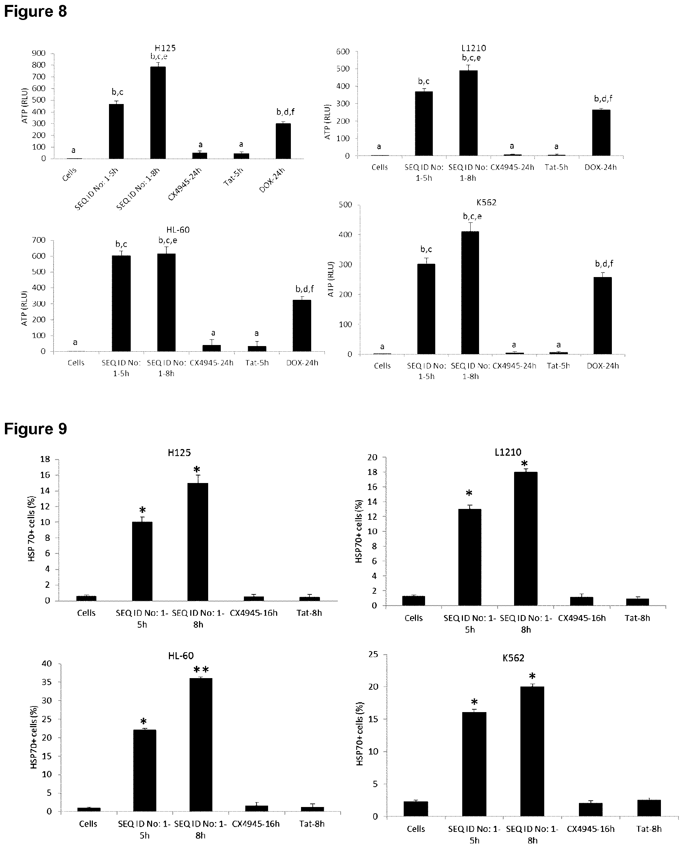

**Figure 8.** Detection of ATP in culture supernatant of several tumor cell lines treated with the peptide identified as SEQ ID NO: 1. The graphs represent ATP levels as relative light units (RLU) detected in each condition. The Bar graphs represent mean $\pm$ standard deviation of three independent replicates. Statistical significance was determined using Kruskal-Wallis test and Dunn's multiple comparisons. Different letters indicate statistically significant differences: *p<0.05.*

**Figure 9.** Surface expression of HSP70 on several tumor cell lines after the peptide identified as SEQ ID NO: 1 treatment. The graphs represent the percentage of HSP70+ cells. The bars represent mean $\pm$ standard deviation of three independent replicates. Statistical differences were determined by one-tail ANOVA, *\*p<0.05;\*\*p<0.01.*

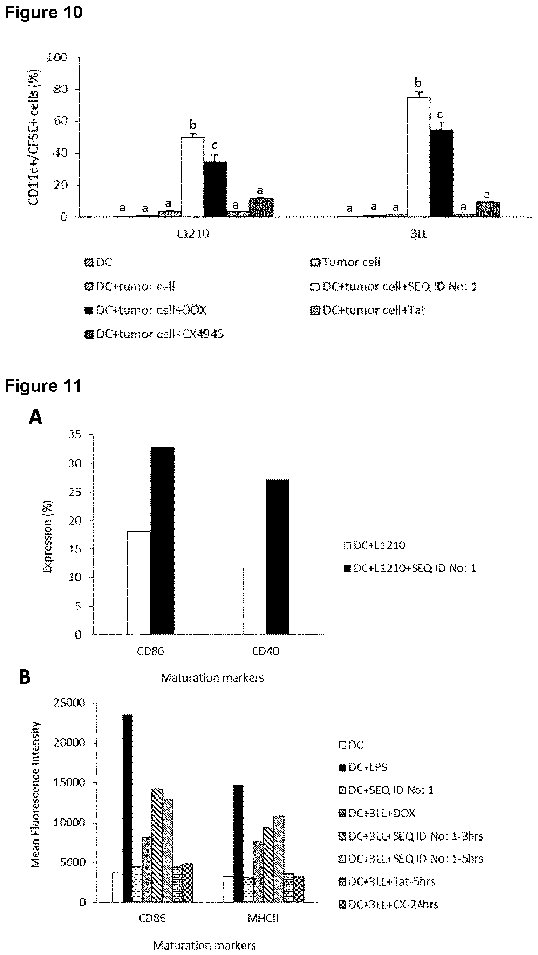

**Figure 10.** Phagocytosis of L1210 and 3LL cell lines by dendritic cells after treatment with the peptide identified as SEQ ID NO: 1. The graph shows percentage of CD11c+/CFSE+ cells. The error bars indicate standard deviation from mean of six replicates of three independent experiments. Different letters indicate statistically significant differences (Fisher's exact test, *p<0.05*).

**Figure 11.** Expression of maturation markers of dendritic cells after co-culture with L1210 and 3LL cells treated with the peptide identified as SEQ ID NO: 1. Bar graphs show expression of maturation markers of dendritic cells after co-culture with L1210 (**A**) and 3LL (**B**) tumor cell lines.

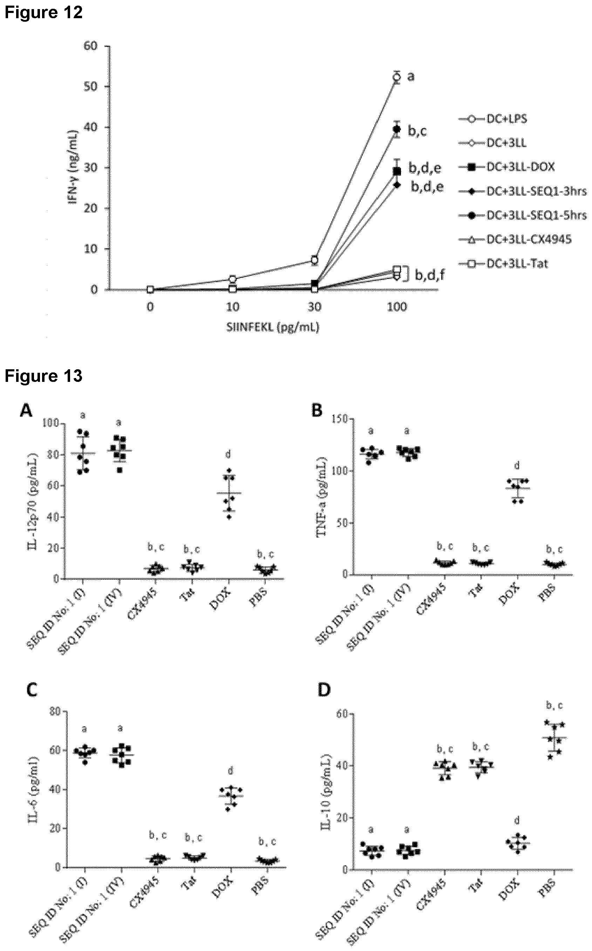

**Figure 12.** *In vitro* activation of OT-I CD8+ cells by dendritic cells. The graph shows IFN-y concentration detected by ELISA in supernatant of culture of dendritic cells co-cultured with tumor cells pre-treated with the peptide identified as SEQ ID NO: 1, CX4945, Tat peptide or DOX for 48 hours. Error bars indicate standard deviation from mean of six replicates of three independent experiments. Different letters indicate statistically significant differences regarding IFN-y concentration (Kruskal-Wallis test and Dunn's multiple comparisons, *p<0.05*). SEQ1: Peptide identified as SEQ ID NO: 1. X axis represents OVA class I peptide concentration of SIINFELK sequence.

**Figure 13.** Quantification of IL-12p70 (**A**), TNF-$\alpha$ (**B**), IL-6 (**C**) e IL-10 (**D**) levels in peripheral blood serum of DBA/2 mice challenged with L1210 cells, after seven days of treatment with the peptide identified as SEQ ID NO: 1. Graphs show cytokine levels of the individual mice (mean ± standard deviation). Different letters indicate statistically significant differences (Kruskal-Wallis test and Dunn's multiple comparisons, *p<0.01*).

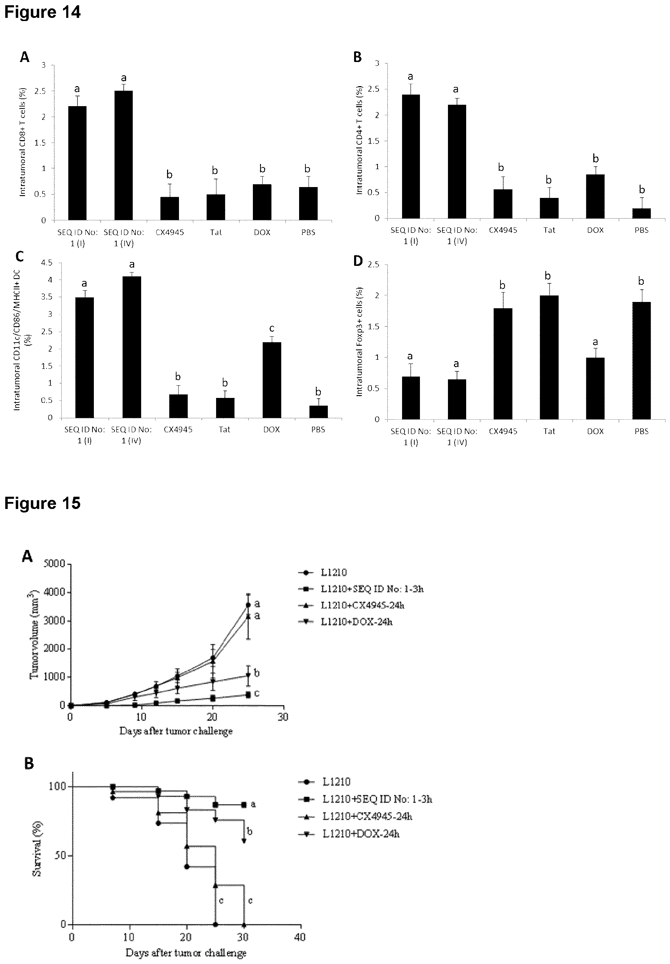

**Figure 14.** Study of different intratumor cell populations seven days after treatment of DBA/2 mice with the peptide identified as SEQ ID NO: 1. Figure show percentage of intratumor CD8+ T cells (**A**), CD4+ T cells (**B**), CD11c/CD86/MHC-II+ dendritic cells (**C**) and Foxp3+ T cells (**D**) in DBA/2 mice treated with the peptide identified as SEQ ID NO: 1, CX4945, DOX or Tat peptide. Bars represent mean ± standard deviation of each group (n=7). Statistical differences were determined by one-tail ANOVA, *p<0.05.*

**Figure 15.** Evaluation of *in vivo* immunogenicity of L1210 cells treated with the peptide identified as SEQ ID NO: 1 in DBA/2 mice. Graphs correspond to tumor growth curve (mean ± standard deviation) (**A**) and survival (Kaplan-Meier curve) (**B**). Different letters indicate statistically significant differences (*p<0.05*) in comparisons made between the groups, using a one-way ANOVA test and Tukey multiple comparison test for tumor volume, and Log-rank test for survival analysis.

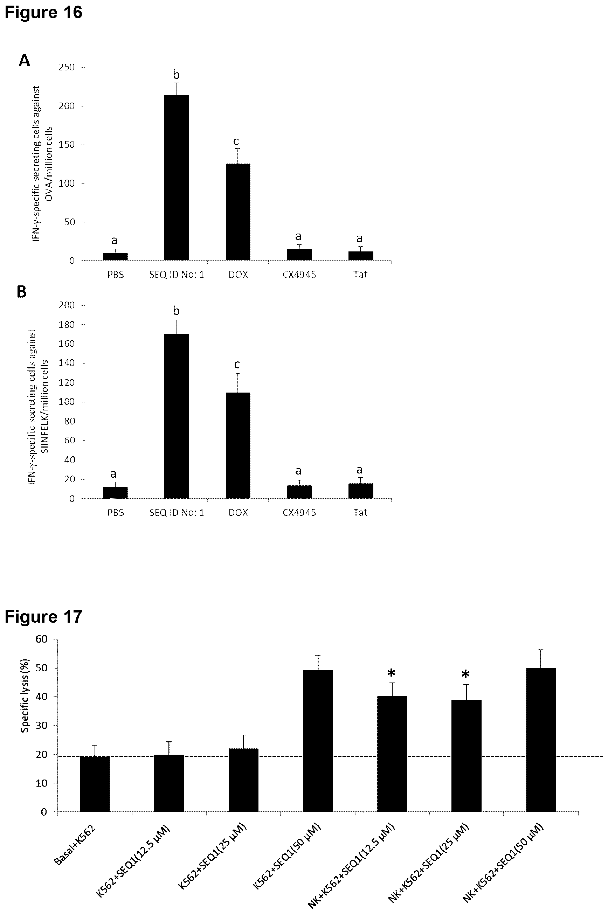

**Figure 16.** Evaluation of cellular immune response by IFN-y secretion against OVA (**A**) and SIINFELK peptide from MHC class I-restricted OVA (**B**) in C57/BL6 mice that were inoculated with 3LL-OVA cells. The results are shown as average number of points per million cells in each group ± standard deviation. Different letters indicate statistically significant differences regarding number of points per million cells (Kuskal-Wallis test and Dunn's multiple comparisons, *p<0.05*).

**Figure 17.** Cytotoxic activity of NK cells on K562 after treatment with the peptide identified as SEQ ID NO: 1. The graph shows mean ± standard deviation of three independent replicates. The asterisk (*) indicates statistically significant differences regarding specific lysis percent with respect to baseline control (one-way ANOVA, Tukey multiple comparison test, *p<0.05*). SEQ1: Peptide identified as SEQ ID NO: 1.

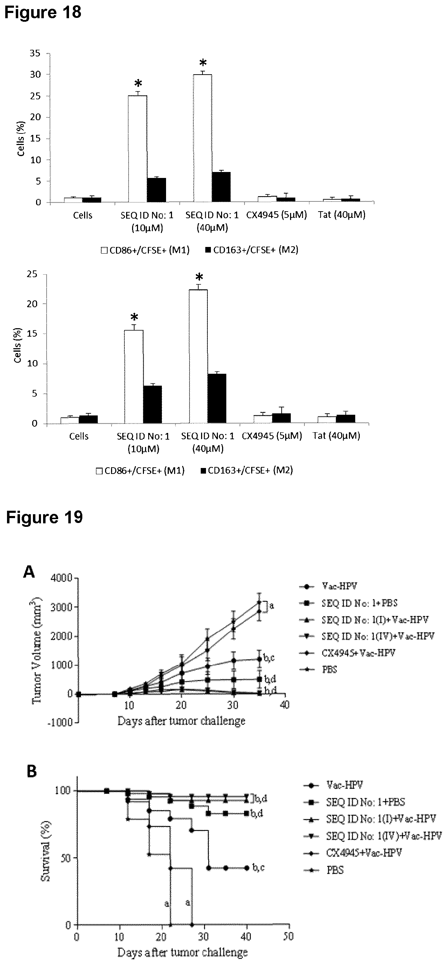

**Figure 18.** *In vitro* evaluation of effect of the peptide identified as SEQ ID NO: 1 on the phagocytic capacity of macrophage subtypes M1 and M2. OCI-AML3 (**A**) and HL-60 (**B**) cells were treated with the peptide identified as SEQ ID NO: 1, CX4945 or Tat. The graph shows mean ± standard deviation of three independent experiments. The asterisk (*) indicates statistically significant differences regarding percentage of CFSE+ cells in each subtype of macrophages evaluated, with respect to cells without treatment (one-way ANOVA, Tukey multiple comparison test, *p<0.05).*

**Figure 19.** Evaluation of antitumor effect of the combination of the peptide identified as SEQ ID NO: 1 and the vaccine candidate CIGB550-E7+VSSP. Figure shows the graphs corresponding to tumor growth curve (mean ± standard deviation) (**A**) and survival (Kaplan-Meier curve) (**B**). Different letters indicate statistically significant differences (*p<0.05*) in comparisons made amount groups, using a one-way ANOVA test and Tukey multiple comparison test for tumor volume, and Log-rank test for survival analysis.

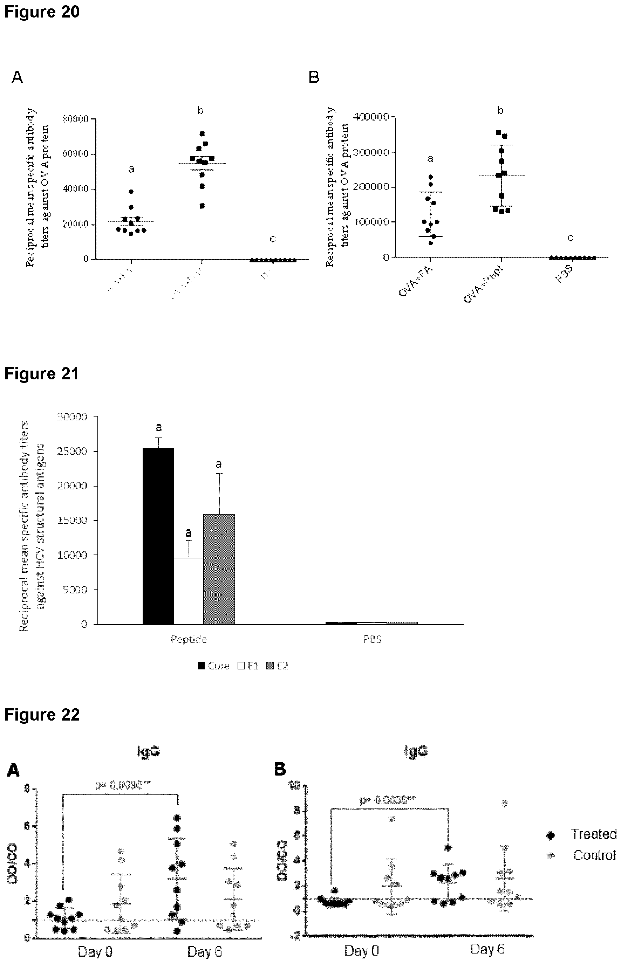

**Figure 20.** Evaluation of humoral response against OVA protein. The results are shown as the reciprocal of mean of antibodies titer against OVA protein, determined by ELISA, on days 21 (**A**) and 42 (**B**) of immunization schedule. Error bars indicate standard deviation of mean of each group titers. Different letters indicate statistically significant differences regarding antibody titers (ANOVA, Tukey multiple comparison test, *p<0.01*).

**Figure 21.** Evaluation of humoral response against Core.120, E 1.340 and E2.680 proteins. Results are shown as the reciprocal of the mean of antibody titers against Core.120, E 1.340 and E2.680 proteins determined by ELISA six days after challenge with a recombinant vaccinia virus that expresses the structural proteins of hepatitis C virus (HCV). Error bars indicate standard deviation of the mean of antibody titers against each protein. "a": denote statistically significant different with regard to control group regarding antibody titers (ANOVA, Tukey multiple comparison test, *p<0.01*).

**Figure 22.** Evaluation of humoral response against nucleocapsid (NP) (**A**) and S protein receptor-binding domain (RBD) (**B**) of SARS-CoV-2 virus. Graphs show IgG antibody levels in samples of patients treated with the peptide identified as SEQ ID NO: 1 in combination with standard therapy and control group treated only with standard therapy. Significance levels *p<0.05* (Wilcoxon test). DO/CO: Optical density of the sample between cut-off value of the assay.

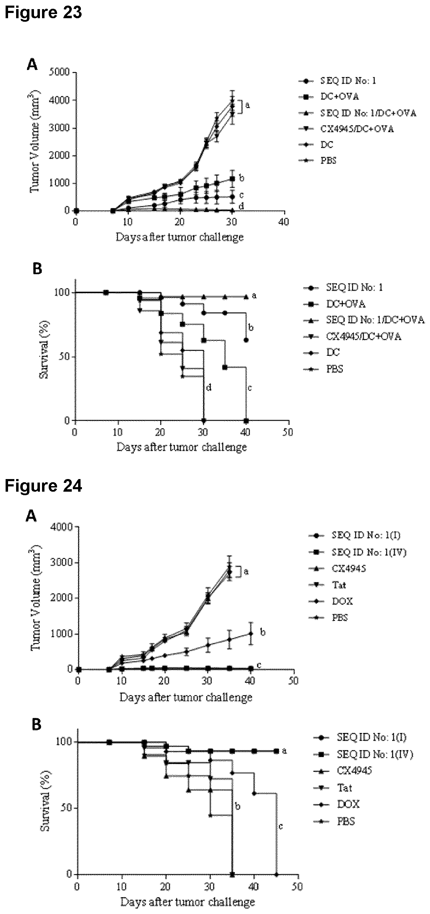

**Figure 23.** Evaluation of the ability of the peptide identified as SEQ ID NO: 1 to enhance the effect of dendritic cell-based treatment. Figure shows the graphs corresponding to tumor growth curve (mean ± standard deviation) (**A**) and survival (**B**). Different letters indicate statistically significant different (*p<0.05*) in comparisons made amount groups using a one-way ANOVA test and Tukey multiple comparison test for tumor volume and Log-rank test for survival analysis.

**Figure 24.** Evaluation of ability of the peptide identified as SEQ ID NO: 1 to enhance the effect of a TIL-based antitumor treatment. Figure shows the graphs corresponding to tumor growth curve (mean ± standard deviation) (**A**) and survival (**B**). Different letters indicate statistically significant different (*p<0.05*) among groups using a one-way ANOVA test and Tukey multiple comparison test for tumor volume and Log-rank test for survival analysis.

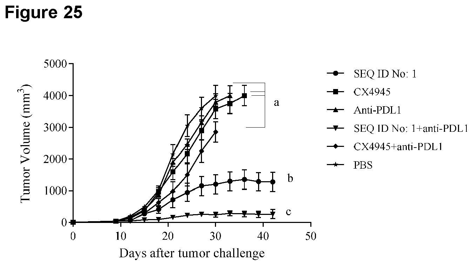

**Figure 25.** Evaluation of ability of the peptide identified as SEQ ID NO: 1 to enhance the antitumor efficacy of anti-PDL1 therapy. The figure shows the graph corresponding to tumor growth curve (mean ± standard deviation).

Different letters indicate statistically significant different (*p<0.05*) among groups using a one-way ANOVA test and Tukey multiple comparison test for tumor volume.

**Examples**

**Example 1. Cell death induced by the peptide identified as SEQ ID NO: 1.**

In order to corroborate whether the peptide identified as SEQ ID NO: 1 was capable of inducing cell death in tumor cell lines, mouse lymphocytic leukemia cells L1210 were treated with 10 $\mu$M peptide identified as SEQ ID NO: 1 for 20 minutes at 4°C and 37°C. After that time, cells were labeled with Annexin V-FITC, which bind to phosphatidylserine residues exposed on the outer membrane of apoptotic cells, and 7-AAD, which only penetrates into dead cells. Finally, samples were analyzed by flow cytometry.

Treatment with the peptide identified as SEQ ID NO: 1 at 37°C elicited an increase of up to 15 folds the percentage of apoptotic cells (Annexin V+/7-AAD-). This increase was not detected when the cells were incubated at 4°C, which could indicate the induction of apoptosis, since this is an energy-dependent process (Figure 1A). Similar results were observed when 3LL murine lung cancer cells were treated with 130 $\mu$M peptide identified as SEQ ID NO: 1 for 3 and 5 hours (Figure 1B).

In *ex vivo* evaluations carried out with samples from patients with acute myeloid leukemia, induction of cell death was also observed when cells were treated with 40 $\mu$M of peptide identified as SEQ ID NO: 1 during 24 hours. This phenomenon was observed in 6 out of 10 patients evaluated (Figure 2). These results demonstrate that the peptide identified as SEQ ID NO: 1 is capable of inducing tumor cell death both *in vitro* and *ex vivo.*

**Example 2. Translocation of CRT and Erp57 induced by the peptide identified as SEQ ID NO: 1.**

CRT exposure at the tumor cell membrane constitutes a phagocytic signal ("eat me" signal) for dendritic cells, which also occurs accompanied by Erp57 expression. Taking this into account, we decide to evaluate whether the peptide identified as SEQ ID NO: 1 was capable of causing the translocation of these molecules in L1210 cells. For this purpose, $2.5 \times 10^5$ cells were seeded in 12-wells plate and treated with 40 $\mu$M peptide identified as SEQ ID NO: 1 for 3 and 5 hours. Subsequently, the cells were collected, washed twice with cold PBS and fixed with 0.2% paraformaldehyde for 5 minutes at 4°C. After Fc receptor blocking (with PBS containing 2% fetal bovine serum (FBS) for 30 minutes at 4°C), the cells were labeled with the primary antibodies for 30 minutes at 4°C, followed by two washes and incubation with a secondary monoclonal antibody conjugated to Alexa 488 in blocking solution for 30 minutes at 4°C. After repeating washes, the cells were analyzed by Flow Cytometry. In addition, the effect of the CK2 inhibitor called CX4945 was also evaluated after treating with 5 $\mu$M for 24 hours. Cells without treatment constituted the negative control of the test.

Treatment with the peptide identified as SEQ ID NO: 1 at the two times evaluated did induce CRT and Erp57 translocation at the cell surface in L1210 cells (Figures 3 and 4). The percentage of CRT+ and Erp57+ cells increased significantly compared to the cells without treatment and those treated with the inhibitor CX4945 (Figures 3 and 4). These results demonstrate that the peptide identified as SEQ ID NO: 1 causes the translocation to the cell surface of molecules that constitute "eat me" signals, which constitutes an essential stimulus for phagocytosis and capture of tumor antigens.

**Example 3. Treatment with peptide identified as SEQ ID NO: 1 decrease the CD47 "don't eat me" signal in tumor cells.**

Recently, a strong association has been reported between anti-phagocytic signals, also known as "don't eat me" signals, with tumor growth and progression. In this sense, we evaluated whether the peptide identified as SEQ ID NO: 1 had any effect on these molecules, specifically on CD47. To do that, two leukemia cell lines were employed: HL60 (ATCC® CCL240™) and OCI-AML3 (DSMZ ACC 582). Cells were maintained in RPMI-1640 medium (Sigma, EEUU) supplemented with 10% FBS (Capricorn Scientific GmbH, Alemania), 2mM L-glutamine and 50 $\mu$g/mL gentamicin (Sigma, EEUU). The experimental design consisted of 8 experimental groups in both cell lines, treated with 40 $\mu$M of peptide identified as SEQ ID NO: 1 for 30 minutes and 3 hours, with untreated cell controls at each times. Three experimental replicates were used per group in 12-well plates, for a total of 24 independent samples as shown in Table 1.

**Table 1.** Experimental design in HL60 and OCI-AML3 cell lines

| Cell line | Treatment | Time | No. replicates |
|-----------|-----------|------|----------------|
| HL60 | Cell control | 30min / 3h | 3/condition |

(continued)

| Cell line | Treatment | Time | No. replicates |
|---|---|---|---|
| HL60 | 40 μM SEQ ID NO: 1 | 30min / 3h | 3/ condition |
| OCI-AML3 | Cell control | 30min / 3h | 3/ condition |
| OCI-AML3 | 40 μM SEQ ID NO: 1 | 30min / 3h | 3/ condition |

After 30 minutes and 3 hours of incubation, culture medium was removed, cells were washed once with PBS buffer and each well was collected in Lysis Buffer (RNeasy Plus Minikit, Qiagen, EU) with 1% β-mercaptoethanol and homogenized. Purification proceeded according to manufacture instructions QiaCube (Qiagen, EEUU). Total ribonucleic acid (RNA) samples were resuspended in nuclease-free water and stored at -70°C until microarray differential gene expression was analyzed. The results were further validated by qPCR. Oligonucleotides used are shown in Table 2. *GAPDH, DDX5* and *ABL1* were employed as reference genes. Reactions were carried out on LightCycler®480II equipment (Roche, Germany) in 96-well plates in SYBR Green Probe II mode.

**Table 2.** Oligonucleotides used in qPCR gene amplification.

| Gene | GenBank Identifier | Oligonucleotide Sequence | Base numbers | Function |
|---|---|---|---|---|
| CD47-F | NM_001777.3 | CCAATGCATGGCCCTCTTCT | 20 | Biomarker |
| CD47-R | | GGGTTCCTCTACAGCTTTCCTA | 22 | |
| GAPDH-F | NM_002046 | GTCCACCACCCTGTTGCTGTAG | 22 | Reference |
| GAPDH-R | | ACTTCAACAGCGACACCCACTC | 22 | |
| DDX5-F | NM_004396 | TAGAGGTCACAACTGCCCGAAG | 22 | Reference |
| DDX5-R | | GGCCATCCCTGAGCTTGAATAG | 22 | |
| ABL1-F | NM_007313.2 | ACGTCTGGGCATTTGGAGTATT | 22 | Reference |
| ABL1-R | | CTCCATGCGGTAGTCCTTCTCT | 22 | |

Its official symbol, identifier in GenBank™ sequence database, sequence of forward (F) and reverse (R) oligonucleotides and number of bases of each are shown.

Fold change of each gene was determined with respect to untreated condition, normalized with the three reference genes *GAPDH, DDX5* and *ABL1*. As shown in Figure 5, treatment with the peptide identified as SEQ ID NO: 1 for 3 hours induced a significant decrease in CD47 messenger RNA (mRNA) levels in both cell lines evaluated. This was observed by both microarrays (Figure 5A) and qPCR (Figure 5B). These results indicate that the peptide identified as SEQ ID NO: 1 modulates the levels of "don't eat me" signals like CD47.

**Example 4. Induction of danger signals by the peptide identified as SEQ ID NO: 1.**

During the immunogenic cell death, expression and release of certain danger signals involved in the activation of an effective antitumor immune response is induced. Taken this into account, we evaluated the ability of the peptide identified as SEQ ID NO: 1 to induce the release of ATP, HSP70 and HMGB-1 in different human and murine tumor cell lines and in serum from patients treated with the peptide.

For ATP, HSP70 and HMGB-1 *in vitro* detection, $6 \times 10^4$ cells were seeded in 24-well plates and treated with: 40 μM peptide identified as SEQ ID NO: 1, 40 μM Tat both for 5 and 24 hours, and 5 μM CX4945 for 24 hours. Cells treated with 5 μM DOX for 24 hours as positive control. Untreated cells and serum from patients taken at zero time constituted the negative controls. ATP secretion was measured by luciferin-based ENLITEN ATP Assay (Promega), HSP70 were stained with an anti-HSP70 antibody (clone C92F3A-5, Enzo Life Sciences) and HMGB-1 release was assessed by enzyme-linked immunosorbent assay (ELISA, IBL International) according to manufacturer's instructions.

Treatment with the peptide identified as SEQ ID NO: 1 for 5 and 24 hours elicited HMGB-1 release to culture medium. Levels detected were statistically higher than those found in untreated cells or treated with either Tat peptide or the CK2 inhibitor, CX4945 (Figure 6). At 24 hours HMGB-1 levels secreted into culture medium by cells treated with the peptide identified as SEQ ID NO: 1 were significantly higher than those of cells treated with DOX. An increase in HMGB-1 levels was observed in the serum of patients treated with the peptide identified as SEQ ID NO: 1 (Figure 7). This was observed in 4 out of 5 patients evaluated, mainly at the sixth week of treatment.

ATP levels also increased significantly in supernatants of tumor cell cultures after treating with the peptide identified as SEQ ID NO: 1 for 5 and 24 hours, compared to untreated cells and those treated with CX4945 (Figure 8). Levels were even higher than those detected after treatment with the classic immunogenic cell death inducer, DOX. Similar results were observed when HSP70 was evaluated. Treatment with the peptide identified as SEQ ID NO: 1 for 5 and 24 hours elicited a significant increase in the HSP70 positive cells percentage, compared to the rest of evaluated conditions (Figure 9). These results demonstrate that the peptide identified as SEQ ID NO: 1, unlike CX4945 and Tat peptide, causes the induction of danger signals involved in the recruitment and activation of immunity cells.

**Example 5. Phagocytosis, maturation and activation of dendritic cells *in vitro* by tumor cells treated with the peptide identified as SEQ ID NO: 1.**

To evaluate whether tumor cells treated with the peptide identified as SEQ ID NO: 1 are capable of stimulating phagocytosis and maturation of dendritic cells, L1210 and 3LL cells were treated with 40 $\mu$M and 130 $\mu$M, respectively, of the peptide identified as SEQ ID NO: 1 in T25 flasks, for 3 hours. For the phagocytosis experiment, tumor cells were previously stained with 1 $\mu$M of Carboxyfluorescein succinimidyl ester (CFSE). After treatment, tumor cells were washed and seeded in U-bottom 96-well plates, 1:2 ration with respect to dendritic cells, for 48 hours. Dendritic cells were differentiated using FLT3, from bone marrow precursors of DBA/2 mice, for co-culture with L1210 cells, and C57/BL6 mice for the case of the experiment with 3LL cells. The co-culture was labeled with anti-CD11c antibody, for the case of phagocytosis experiments, or with anti-CD11c, anti-CD86, anti-MHCII and anti-CD40 antibodies for dendritic cells maturation experiments. Untreated cells constituted the negative control of experiment. DOX-treated cells were used as positive control. The effect of Tat peptide and the CK2 inhibitor called CX4945 on the stimulation of phagocytosis was also evaluated.

Additionally, the ability of activating CD8+ T cells *in vitro* by these dendritic cells matured with peptide-treated 3LL cells was evaluated. For this purpose, dendritic cells previously co-incubated with tumor cells, under the conditions described above, were washed and loaded with the SIINFELK peptide from OVA, MHC class I restricted, at different concentrations (0, 10, 30 and 100 pg/mL) during three hours. After that time, they were washed again and co-incubated with RagOT-I cells in a 1:1 ratio for 48 hours. IFN-$\gamma$ production was finally quantified by ELISA. RagOT-I cells co-incubated with dendritic cells previously incubated with untreated 3LL cells constituted the negative control of experiment.

As shown in Figure 10, treatment with the peptide identified as SEQ ID NO: 1 stimulated phagocytosis of tumor cell by bone marrow derived dendritic cells. Percentage of CD11c+/CFSE+ cells in the case of the co-culture of dendritic cells with tumor cells treated with the peptide identified as SEQ ID No:1 was significantly higher than the rest of evaluated conditions (*p<0.05*). Under these same conditions, induction of dendritic cells maturation was observed by an increase in CD40, CD86 and MHC-II markers (Figure 11).

Dendritic cells matured with peptide- and DOX-treated 3LL cells, were capable to activate CD8+ OT-I cells as determined by an increase in IFN-y production, which was significantly higher than negative control and cells treated with CX4945 and Tat peptide (Figure 12). These results demonstrate that treatment with the peptide identified as SEQ ID NO: 1 induces the expression of molecules that stimulate phagocytosis and maturation of dendritic cells, as well as an increase in their capacity to activate CD8+ T cells. This effect could indicate the initiation of an antitumor response induction.

**Example 6. Evaluation of the effect of the peptide identified as SEQ ID NO: 1 on tumor microenvironment in DBA/2 mice.**

To study whether the peptide identified as SEQ ID NO: 1 was capable of modulating the tumor microenvironment, female DBA/2 mice, 6-8 weeks old, and between 17-19 grams were inoculated with $0.5\times10^6$ L1210 cells, subcutaneously, into right flank (inguinal inoculation). Seven days after tumor challenge, 42 mice with palpable and non-measurable tumor were selected, which were random assigned to six groups of seven animals each. The mice received the treatment as shown in Table 3. Treatment with the peptide identified as SEQ ID NO: 1 (20 mg/kg) was administered intratumorally and intravenously, in a volume of 50 $\mu$L and 100 $\mu$L, respectively, with a daily inoculation for five days. DOX (10 mM) and Tat peptide (20 mg/kg), were administered intratumorally, in a final volume of 50 $\mu$L, a daily inoculation for five days. The compound CX4945 (75 mg/kg) was administered in 25 mM $NaH_2PO_4$ buffer, twice a day, orally, for five days. Seven days after the end of the treatment, the mice were euthanized to obtain the tumors (day 18 of the procedure). Before sacrificing the mice, blood samples were obtained for the cytokines (IL-6, IL-10, IL-12p70, TNF-$\alpha$) detection by ELISA. Tumors were cut into small pieces and transferred to gentleMACS tubes with RPMI medium, supplemented with an enzyme cocktail from Tumor Dissociation kit (Miltenyi Biotech, Germany). Tumor dissociation was performed using the gentleMACS™ Dissociator kit and a tumor dissociation kit (Miltenyi Biotech, Germany). The digested suspension was filtered, centrifuged and labeled with specific antibodies against CD8, CD4, Foxp3, CD11c, CD86 and CD40.

**Table 3.** Experimental design summary.

| Group | Test substance | Procedure day | | | | | |
|---|---|---|---|---|---|---|---|
| | | Day 0 | Day 7 | Day 8 | Day 9 | Day 10 | Day 11 |
| 1 | Peptide SEQ ID NO: 1 | R | I | I | I | I | I |
| 2 | Peptide SEQ ID NO: 1 | R | IV | IV | IV | IV | IV |
| 3 | CX4945 | R | O | O | O | O | O |
| 4 | Tat | R | I | I | I | I | I |
| 5 | DOX | R | I | I | I | I | I |
| 6 | PBS | R | I | I | I | I | I |
| **R:** Tumor challenge, **I:** intratumoral administration, **IV:** intravenous, **O:** oral administration twice daily. | | | | | | | |

Treatment with the peptide identified as SEQ ID NO: 1 administered both intratumoral and systemically, induced a significant increase of IL12p70, TNF-$\alpha$ and IL-6 levels in peripheral blood, compared to the rest of the groups. Otherwise, peptide treatment significantly reduced IL-10 levels. This modulation in cytokine levels was not observed when mice were treated with Tat, DOX or CX4945 (Figure 13).

Similarly, treatment with the peptide identified as SEQ ID NO: 1, by the two administration routes caused a significant increase in the intratumoral levels of CD8+ and CD4+ T cells, as well as *in situ* maturation of dendritic cells, as determined by increase of CD86 and MHC-II maturation markers. Additionally, a significant decrease in intratumoral Foxp3+ T cell levels was observed after treatment with the peptide identified as SEQ ID NO: 1 and DOX, obtaining a greater reduction after application of the peptide. This effect was not observed when mice were treated with Tat or CX4945 (Figure 14). These results indicate that treatment with the peptide identified as SEQ ID NO: 1 is capable of reverting the immunosuppressive tumor environment to immunostimulatory, with a marked increase in dendritic cells maturation and recruitment of effector cells.

**Example 7. Evaluation of the vaccine effect of the inoculation of tumor cells treated with the peptide identified as SEQ ID NO: 1 in DBA/2 mice.**

In order to explore whether the cell death induced by the peptide identified as SEQ ID NO: 1 had any immunomodulatory effect, a vaccination schedule was performed in mice with tumor cells previously treated with the peptide identified as SEQ ID NO: 1. For this purpose, $1 \times 10^6$ L1210 cells treated *in vitro* with 40 $\mu$M of the peptide identified as SEQ ID NO: 1, or time-matched untreated cells during 3 hours were subcutaneously injected in 0.1 mL in the inguinal area into right flank of 6-8 weeks female DBA/2 mice. After seven days, $1 \times 10^6$ live L1210 cells were inoculated on opposite flank, and tumor growth and volume were evaluated until day 45. Tumors were measured with Vernier calipers, three times a week, tumor volume was estimated using the formula: Volume=width$^2$ (mm) $\times$ length/2 (mm). As a positive control, cells treated with 15 $\mu$M DOX for 24 hours were used for vaccination. A group vaccinated with 5 $\mu$M CX4945-treated cells for 24 hours was also included. In total 4 groups were formed of 10 mice each group.

As showed in Figure 15, mice vaccinated with tumor cells treated with the peptide identified as SEQ ID NO: 1, exhibited a clear tumor growth delay which can be due to the induction of an antitumor immune response. The volume of tumors in this group was lower than the rest of the groups, a difference that became statistically significant (Figure 15A, *p<0.05*). Importantly, in the group inoculated with cells treated with the peptide identified as SEQ ID NO: 1, seven out of ten mice remained tumor-free until the end of the study, which was significantly superior to the rest of the conditions evaluated (Figure 15B, *p<0.01*). These results corroborate that the cell death induced by the peptide identified as SEQ ID NO: 1 is immunogenic as it generates an immune response that prevents the growth of a subsequent tumor challenge. Cells treated with CX4945 did not induce any vaccine effect.

**Example 8. Cellular immune response generated by tumor cells treated with the peptide identified as SEQ ID NO: 1 in C57/BL6 mice.**

The induction of cellular immune response by tumor cells treated with the peptide identified as SEQ ID NO: 1 was evaluated. For this purpose, $1 \times 10^6$ 3LL-OVA cells treated or untreated *in vitro* with one of following variants: a) 40 $\mu$M of the peptide identified as SEQ ID NO: 1 for three hours, b) 15 $\mu$M DOX for 24 hours, c) 5 $\mu$M CX4945 and d) 40 $\mu$M Tat for three hours, were inoculated subcutaneously, in inguinal area of the right leg of 6-8 weeks female C57/BL6 mice in a volume of 0.1 mL. After 5 days, five mice from each group were euthanized to get lymphoid nodules which were subsequently macerated to acquire the cells.

IFN-γ secretion was evaluated by enzyme linked immunospot assay (ELISPOT), following the procedure described below. 96-well microplates with nitrocellulose membranes (Millipore, Bedfors, MA, USA) were coated with 100 μL/well of murine anti- IFN-y monoclonal antibody (BD Biosciences, Canada) at a concentration of 5 μg/mL in PBS, for 16 hours at 4°C. After three washes with PBS the plates were blocked with RPMI medium supplemented with 10% FBS, for 1 hour at 37°C in a humid atmosphere with 5% $CO_2$. A total of $2\times10^5$ cells/well were stimulated, in duplicate, with OVA protein and SIINFELF peptide from OVA, at a final concentration of 6 μg/mL, in a final volume of 0.2 mL. Concanavalin A at 5 μg/mL, in RPMI medium with 10% FBS, was used as positive control. The plates were incubated for 48 hours at 37°C, in humid atmosphere of 5% $CO_2$. After incubation, a standard ELISPOT assay was performed (Vázquez-Blomquist D et. al. (2002) Viral Immunol. 5(2):337-356). Counting of spots was carried out in automatic counter (Immunospot Analyzer, Cellular Technology Ltd., Shaker Heights, OH, USA). Cells incubated with RPMI medium with 10% FBS were taken as negative controls.

Results were considered positive when the number of spots was at least three times higher than the average number of spot in the negative control group and there were at least three specific spots (value obtained after subtracting the number of spots from an individual animal in a stimulation condition minus the number of spots of the same in the non-stimulation condition) per $10^6$ cells.

As shown in Figure 16, inoculation of 3LL-OVA cells treated with the peptide identified as SEQ ID NO: 1 elicited a response of IFN-γ-secreting effector cells that was significantly higher than the rest of the groups. Tumor cells treated with CX4945 and Tat were not able to generate this type of response. These results demonstrate that the peptide identified as SEQ ID NO: 1, and not another CK2 inhibitor, such as CX4945, or Tat peptide, induces a type of cell death capable of generating an effective CD8+ T lymphocyte response.

**Example 9. Evaluation of the effect of peptide identified as SEQ ID NO: 1 on tumor cell susceptibility towards NK activity.**

The release of danger signals such as HSP70 and CRT has been reported to be involved in increasing the susceptibility of tumor cells toward cytotoxic activity of NK cells. Therefore, the effect of the peptide identified as SEQ ID NO: 1 on the susceptibility of tumor cells toward NK cytotoxic activity was evaluated. To do that, the classic target cells to NK activity, K562 cells, were employed. Target cells were labeled with $Cr^{51}$ (approximate specific activity: 100 μCi/μg) by incubation for 70 minutes at 37°C. Subsequently, target cells were washed three times with RPMI medium supplemented with 2% FBS and seeded in 96-well plates, at a target:effector ratio of 1:25. On the other hand, NK cells were isolated from peripheral blood mononuclear cells (PBMC) of healthy donors, by negative selection, using magnetic beads (Miltenyi Biotec). Thus, CD56+CD3-NK cells were obtained with more than 96% purity. The peptide identified as SEQ ID NO: 1 was added to the co-culture at 12.5; 25 and 50 μM. After 4 hours of incubation, supernatant was collected for the measurement of $Cr^{51}$ release using a scintillation counter. The percentage of specific lysis was calculated as follows:

$$\% \ specific \ lysis = \frac{CPM \ sample - CPM \ spontaneou}{CPM \ total - CPM \ spontaneous} * 100$$

Target cells without coincubation with effector cells were employed as spontaneous lysis control. Target cells with the peptide identified as SEQ ID NO: 1 constitute the toxicity control of the peptide.

As shown in Figure 17, presence of the peptide identified as SEQ ID NO: 1 in the co-culture of K562 cells with NK cells enhanced the cytotoxic capacity of NK cells. The 12.5 and 25 μM doses significantly increased the percent of specific lysis compared to baseline cytotoxicity. The peptide 50 μM dose showed toxicity to target cells, so in that case it cannot be assured that the observed lysis is due to the effect to NK cells. Data from these experiments indicate that the peptide identified as SEQ ID NO: 1 is capable of sensitizing tumor cells towards direct action of NK cells, which help to generate a diverse and effective immune response.

**Example 10. Evaluation of the effect of peptide identified as SEQ ID NO: 1 on tumor cell susceptibility towards macrophage cytotoxicity.**

Treatment with the peptide identified as SEQ ID NO: 1, as described in examples 2 and 3, is capable of increasing "eat me" signals and decreasing "don't eat me" signals. Therefore, it fostered to evaluate whether the treatment with the peptide identified as SEQ ID NO: 1 facilitate phagocytosis of tumor cells by macrophages, and whether this is performed by M1 or M2 macrophages. For that purpose, HL-60 and OCI-AML3 leukemia tumor cell lines were previously stained with 0.5 $\mu$M CFSE and treated with 10 or 40 $\mu$M of the peptide identified as SEQ ID NO: 1, for five hours, 5 $\mu$M CX4945 for 24 hours or 40 $\mu$M Tat during 5 hours. Subsequently, cells were washed and co-cultured with each type of M1 and M2 macrophages, at a 1:1 ratio for three hours. Different types of macrophages were obtained from PBMC of healthy donors by Ficoll™ gradient. Monocytes were purified by positive selection, using CD 14 beads with an MS column (Miltenyi Biotech, Germany), obtaining 96% purity. Monocytes were seeded at $0.25 \times 10^6$ cells/mL/well, in 12-wells plates and were induced with 100 ng/mL of GM-CSF, for seven days, and with 50 ng/mL of IFN-y for last 24 hours, to obtain M1 macrophages. M2 macrophages were induced with 50 ng/mL of M-CSF for seven days and 100 ng/mL of IL-10 for last 24 hours. Subsequently, supernatants were collected and cells were stained with anti-CD33 (to determine corresponding macrophages population), anti-CD86 (to determine M1 population) and anti-CD 163 (to determine M2 population).

Pretreatment of OCI-AML3 and HL-60 tumor cells with the peptide identified as SEQ ID NO: 1, both at IC50 dose (40 $\mu$M) and suboptimal dose (10 $\mu$M), increased their susceptibility to be phagocytosed by M1 macrophages and not M2 macrophages (Figure 18). The phagocytosis increase became statistically significant regarding to co-culturing macrophages with untreated tumor cells. This effect was not observed when tumor cells were treated with CX4945 or Tat. These results demonstrate that reduction in "don't eat me" signals by the peptide identified as SEQ ID NO: 1, and not by CX4945 or Tat, enhance the susceptibility of tumor cells towards M1 but not M2 macrophages.

**Example 11. Antitumor effect of the peptide identified as SEQ ID NO: 1 combined with the vaccine candidate CIGB550-E7+VSSPs in C57BL6 mice (TC-1 therapeutic scenario).**

Taken into account the capacity of the peptide identified as SEQ ID NO: 1 for inducing immunogenic cell death, the antitumor response generated by the peptide identified as SEQ ID NO: 1 combined with the CTL vaccine candidate CIGB550-E7+VSSPs (Granadillo M. et al., (2019) Vaccine 37(30):3957-3960), was evaluated. The vaccine candidate contains the recombinant fusion protein CIGB550-E7 based on the fusion of a Human Papillomavirus (HPV)-E7 mutein to the cell-penetrating peptide CIGB550, formulated with synthetic NAcGM/VSSP adjuvant.

For that purpose, 6-8 weeks old C57/BL6 female mice were subcutaneously injected with $5 \times 10^4$ TC-1 cells into right flank (inguinal inoculation). Seven days after tumor challenge, 48 mice with palpable and non-measurable tumor were randomly divided into six groups with 10 mice per group. Mice received different treatment as shown in Table 4. Treatment with the peptide identified as SEQ ID NO: 1 (at 40 mg/kg) was administered intratumoral and intravenously, in a final volume of 50 $\mu$L and 100 $\mu$L, respectively. CX4945 (at 75 mg/kg) was administrated in 25 mM $NaH_2PO_4$, twice daily orally. The vaccine candidate CIGB550-E7+VSSPs was injected subcutaneously into right flank in a final volume of 0.2 mL. Tumor growth was followed up and tumor volume was determined and calculated as described in Example 7.

Results obtained showed that the peptide identified as SEQ ID NO: 1, injected both intratumoral and systemically prior to administration of vaccine, is capable of enhancing antitumor effect of CIGB550-E7+VSSPs vaccine candidate, as determined by tumor growth control and mice survival (Figure 19). This data support the use of the peptide identified as SEQ ID NO: 1 to enhance the effect of antitumor vaccines.

**Table 4.** Experimental design summary.

| Groups | Test substance | Days and procedures | | | | | | | | | | | | |
| | | Day 0 | Day 7 | Day 8 | Day 9 | Day 10 | Day 11 | Day 14 | Day 21 | Day 22 | Day 23 | Day 24 | Day 25 | Day 28 |
|---|---|---|---|---|---|---|---|---|---|---|---|---|---|---|
| **1** | CIGB550-E7+VSSPs | **R** | **SC** | - | - | - | - | **SC** | **SC** | - | - | - | - | **SC** |
| **2** | Peptide + PBS | **R** | **I** | **I** | **I** | **I** | **I** | - | - | - | - | - | - | - |
| | | | - | - | - | - | - | - | **SC** | - | - | - | - | **SC** |
| 3 | Peptide + CIGB550-E7+VSSPs | **R** | **I** | **I** | **I** | **I** | **I** | **-** | - | - | - | - | - | - |
| | | | - | - | - | - | - | - | **SC** | - | - | - | - | **SC** |
| **4** | Peptide + CIGB550-E7+VSSPs | **R** | **IV** | **IV** | **IV** | **IV** | **IV** | - | - | - | - | - | - | - |
| | | | - | - | - | - | - | - | **SC** | - | - | - | - | **SC** |
| **5** | CX4945 + CIGB550-E7+VSSPs | **R** | **O** | **O** | **O** | **O** | **O** | **O** | **O** | **O** | **O** | **O** | **O** | **O** |
| | | | - | - | - | - | - | - | **SC** | - | - | - | - | **SC** |
| **6**[*] | PBS | **R** | **I** | **I** | **I** | **I** | **I** | - | - | - | - | - | - | - |

**Peptide:** SEQ ID NO: 1 peptide, **R:** tumor challenge, **I:** intratumoral immunization, **IV:** intravenous immunization, **SC:** subcutaneous immunization, **O:** oral administration twice daily.

EP 4 265 268 A2

**Example 12. Evaluation of the capacity of the peptide identified as SEQ ID NO: 1 to enhance a B cell immune response.**

*Induction of antibodies against OVA protein*

Taken into account the ability of the peptide identified as SEQ ID NO: 1 to induce immunogenic cell death, it was decided to evaluate its capacity to enhance a B cells immune response. For this, 30 female BALB/c mice, eight weeks old and weighing 18-20 grams were divided into three groups of 10 mice per group. The first group was immunized with 10 $\mu$g OVA protein formulated in aluminum phosphate. The second group received 10 $\mu$g OVA protein and 100 $\mu$g of the peptide identified as SEQ ID NO: 1. The third group constituted the control group of the study that was inoculated only with Aluminum Phosphate. The OVA administration was made on days 0, 14 and 28, subcutaneously, in a final volume of 100 $\mu$L. On days 21 and 42 blood samples were obtained to evaluate the induction of IgG response against OVA protein.

As shown in Figure 20, all mice immunized with OVA protein elicited specific antibodies against this protein. Highest antibody titers were induced in the group immunized with OVA protein formulated with the peptide identified as SEQ ID No:1, which were significantly higher than those obtained in the rest of study groups. These results suggest that the peptide identified as SEQ ID No:1 is capable of enhancing a B cells immune response.

*Induction of antibodies against HCV antigens in a subrogate virus model*

Taking into account the results observed in OVA protein formulated with the peptide identified as SEQ ID NO: 1, the ability of this peptide to enhance specific antibody response against viral antigens during an *in vivo* infection, was evaluated. For that purpose, 14 female BALB/c mice, 8 weeks old and weighing 18-20 grams, were challenged with a recombinant vaccinia virus that compresses the structural Hepatitis C virus (HCV) protein (Alvarez-Lajonchere et al. *Biotecnología Aplicada* 2007; 24(3)). The virus was inoculated intraperitoneally at a dose of $10^6$ plaque-forming units (pfu) in 200 $\mu$L of PBS. The following day mice were divided into two groups of seven mice per group. The mice of the first group were treated with 3 mg/kg of the peptide identified as SEQ ID NO: 1 and the second group was inoculated with PBS, both intraperitoneally on days: 1, 2, 3 and 4 of the study. On sixth day after the viral challenge a total blood extraction was carried out to evaluate humoral immune response against HCV structural proteins.

The treatment with the peptide identified as SEQ ID NO: 1 enhanced the induction of specific antibodies against Core. 120, E 1.340 and E2.680 protein antigens. Antibody titers were significantly higher than those of the control group (Figure 21).

*Induction of antibodies in patients infected by anti-SARS-CoV-2 treated with the peptide identified as SEQ ID NO: 1*

A randomized phase I/II clinical trial in 20 SARS-CoV-2 infected patients treated with the peptide identified as SEQ ID NO: 1, was performed. Ten patients were treated intravenously with 2.5 mg of the peptide per kg of body weight, once a day, for five consecutive days along with the standard therapy consisting of Kaletra™, Chloroquine and IFN-$\alpha$2b. On the other hand, control group enrolled 10 patients that only received the standard therapy. Specific anti-NP and anti-RBD IgG levels were determined by using an in home ELISA.

As shown in Figure 22, a significant increase of IgG antibody levels against NP and RBD of SARS-CoV-2 virus was detected after treatment with the peptide identified as SEQ ID NO: 1. In the control group, no significant differences were observed between the levels of antibodies detected in both evaluated times. These results suggest that the peptide identified as SEQ ID NO: 1 is capable of enhancing a specific humoral immune response against viral antigens during a viral infection.

**Example 13. Enhancement of the effect of dendritic cell vaccine by the peptide identified as SEQ ID NO: 1.**

Currently, one of the potentialities in cancer immunotherapy is constituted by dendritic cell-based vaccines. Taking this into account, it was explored whether the peptide identified as SEQ ID NO: 1 was capable of enhancing the effect of a dendritic cell vaccine. For that purpose, C57/BL6 mice were euthanized to obtain spleens, which were macerated and digested with collagenase and DNAse. Low density mononuclear cells were separated using a gradient medium density. Dendritic cells were purified by murine Pan-DC magnetics beads mediated cell drawing (Miltenyi Biotech, Bergisch-Gladbach, Germany), according to manufacturer's recommendations. Dendritic cells were obtained with more than 90% purity as determined by cytometric analyzed of CD 11c expression. The purified cells were loaded with OVA protein (Sigma-Aldrich) (100 $\mu$g/mL) in IMDM medium, supplemented with 10% FBS for 16 hours at 37°C. Non-adherent dendritic cells, loaded with OVA protein (DC+OVA) were collected for inoculation into the animals. Dendritic cells alone were used as negative control. To evaluate whether the peptide identified as SEQ ID NO: 1 is capable to enhance the effect of DC+OVA in a murine model, $5\times10^5$ 3LL-OVA cells were inoculated into 6-8 week C57/BL6 female mice with 17-19 grams of body weight. These 3LL-OVA cells were subcutaneously injected, into right flank, in 50 $\mu$L final volume. Seven days after tumor challenge, 42 mice with palpable and non-measurable tumors were selected, which were randomly divided into six groups with seven mice per group. Animals received different treatments as shown in Table 5. Treatment with the peptide identified as SEQ ID NO: 1 (40 mg/kg) was intratumorally applied in 50 $\mu$L of final volume. CX4945 (75 mg/kg) was orally administered in 25 mM $NaH_2PO_4$ buffer twice daily. The administration of $5\times10^5$ dendritic cells loaded or unloaded with OVA protein was carried out intratumorally, in 50 $\mu$L of final volume. Tumor growth was followed up during time and tumor volume determined and calculated as described in Example 7. The survival of animals was recorded until the tumor volume reached 4000 $mm^3$, time at which animals were euthanized by cervical dislocation. Throughout the experimentation, mice were kept in a pathogen-free environment and the procedures were conducted according to institutional guidelines.

**Table 5.** Experimental design summary.

| Groups | Test substance | Procedure day | | | | | |
|---|---|---|---|---|---|---|---|
| | | Day 0 | Day 3 | Day 4 | Day 5 | Day 6 | Day 7 |
| **1** | SEQ ID NO: 1 peptide | R | I | I | I | I | I |
| **2** | DC+OVA | R | I | - | - | - | - |
| **3** | SEQ ID NO: 1 peptide/DC+OVA | R | I | I | I | I | I |
| **4** | CX4945/DC+OVA | R | O | O | O | O | O |
| **5** | DC | R | I | I | I | I | I |
| **6** | PBS | R | I | I | I | I | I |
| **R:** tumor challenge, **I:** intratumoral immunization, **O:** twice daily oral administration. | | | | | | | |

As shown in Figure 23, the treatment based on the peptide identified as SEQ ID NO: 1 combined with dendritic cells vaccine elicited tumor growth delay in C57/BL6. Tumor volume in this group was significantly lower than the rest of groups (Figure 23A). Significant differences were also observed regarding survival of animals treated with the combination, with respect to the rest of groups (Figure 23B). These results demonstrate that the peptide identified as SEQ ID NO: 1 is capable of enhancing the effect of dendritic cells-based vaccine.

**Example 14. Evaluation of the capacity of the peptide identified as SEQ ID NO: 1 to enhance the effect of an antitumor treatment based on tumor-infiltrating lymphocytes**

The used of TILs for cancer treatment is one of the novel immunotherapeutic strategies currently in application. According to previously obtained results (Example 6), the treatment with the peptide identified as SEQ ID NO: 1 increases the tumor infiltration of CD8+ T cells. Taking this into account, it was decided to evaluate whether the peptide identified as SEQ ID NO: 1 is capable of enhancing the antitumor effect of TILs in a cancer murine model. For that purpose, 6-8 weeks old DBA/2 female mice with 17-19 grams of body weight were subcutaneously inoculated with $5\times10^4$ L1210 cells in right flank (inguinal administration). Seven days after tumor challenge, 42 animals with palpable and non-measurable tumors were randomized into six groups with seven mice per group. The different treatments were administrated as shown in Table 3, Example 6. Seven days after the end of the treatment, mice were euthanized to obtain the tumors. They were cut into small pieces with an approximate size of 1-3 $mm^3$ and seeded in 24-well plates in RPMI medium supplemented with 10% FBS and 2000 IU/mL recombinant murine IL-2. The cultures were expanded in 12-well plates. TILs from fragments of the same tumor were joined and counted. $5\times10^6$ TILs were intravenously inoculated into other DBA/2 mice that were subsequently challenged with $5\times10^4$ L1210 cells at 24 hours. Tumor growth was followed up during the time and tumor volume was measured and calculated as described in Example 7.

As shown in Figure 24, TILs from mice treated with the peptide identified as SEQ ID NO: 1, both intratumorally and systemically, elicited antitumor activity after challenge. Tumor volume mean was significantly lower than the rest of the groups (Figure 24A). Significant differences were also observed in terms of survival of animals treated with TILs from mice treated with the peptide identified as SEQ ID NO: 1, with respect to those from mice treated with DOX, CX4945 or Tat (Figure 24B). These results evidence that the peptide identified as SEQ ID NO: 1 is capable of enhancing the antitumor effect of TIL-based vaccine.

**Example 15. Evaluation of the capacity of the peptide identified as SEQ ID NO: 1 to modulate the levels of immune checkpoint PDL1.**

*PDL1 increase in H460 and A549 cells after the* in vitro *treatment with the peptide identified as SEQ ID NO: 1*

Immune checkpoints are currently an important target in cancer immunotherapy, due to their immunosuppressive role during the course of this disease. Taking this into account, effect of the peptide identified as SEQ ID NO: 1 on modulation of PDL1 levels in two non-small cell lung cancer cell lines: A549 and H549, was evaluated. For that, $5 \times 10^4$ cells were seeded in 24-well plates. The day after, cells were treated with 30 $\mu$M peptide identified as SEQ ID NO: 1; 5 $\mu$M CX4945 or 30 $\mu$M Tat peptide, for 24 hours. After treatment, cells were collected, staining with an anti-PDL1 antibody and analyzed by flow cytometry. Table 6 shows percentage of PDL1 positive cells and mean fluorescence intensity (MFI). Treatment with the peptide identified as SEQ ID NO: 1 elicited a significant increase of PDL1 expression in both cell lines. Differences were not observed when cells were treated with CX4945 or Tat peptide.

**Table 6.** Percentage of PDL1 positive cells and mean fluorescence intensity.

| | H460 | | A549 | |
|---|---|---|---|---|
| **Conditions** | % PDL1$^+$ cells | IFM | % PDL1$^+$ cells | IFM |
| Untreated cells | 26.5 | 16.56 | 30.2 | 17.5 |
| Cells treated with peptide SEQ ID NO: 1 | 86.5** | 45.0* | 90.3** | 48.3* |
| Cells treated with CX4945 | 25.2 | 15.23 | 29.6 | 16.2 |
| Cells treated with Tat | 27.3 | 15.9 | 27.9 | 17.0 |
| Isotype control | 0.01 | 2.1 | 0.06 | 2.2 |
| *, Indicates statistically significant differences (Kruskal-Wallis test and Dunn's multiple comparisons; *, $p<0.05$; **, $p<0.01$). | | | | |

These results suggest that is possible to use the combination of the peptide identified as SEQ ID NO: 1 with PDL1 immune checkpoint inhibitors.

*Evaluation of the capacity of the peptide identified as SEQ ID NO: 1 to enhance the effect of the anti-PDL1 therapy in a murine model of lung cancer.*

Taking into account the results obtained in the *in vitro* test, it was decided to evaluate whether the use of the peptide identified as SEQ ID NO: 1 improved the antitumor efficacy of anti-PDL1 therapy in a murine model of lung cancer. For that, $2 \times 10^5$ Lewis Lung Carcinoma cells were subcutaneously implanted (day 0) in 6-8 weeks old C57/BL6 female mice with 17-19 grams of body weight. Nine days after the tumor challenge, 42 animals with 40 mm$^3$ tumors were randomized into six groups with seven mice each. The different treatments were administrated as shown in Table 7. CX4945 (75 mg/kg) was orally administered in 25 mM NaH$_2$PO$_4$ buffer twice daily. 40 mg/kg of the peptide identified as SEQ ID NO: 1 and 100 $\mu$g anti-PDL1 antibody (clone 10F.9G2, isotype rat IgG2b kappa) were intraperitoneally applied. Tumor volume and body weight were determined 3 times a week for 40 days. Tumor volume was measured and calculated as described in Example 7.

**Table 7.** Experimental design summary.

| Groups | Test substance | Days of test substance administration | Route of administration |
|---|---|---|---|
| 1 | Peptide SEQ ID NO: 1 | 9, 10, 11, 12, 13 | IP |
| 2 | CX4945 | 9, 10, 11, 12, 13 | O |
| 3 | Anti-PDL1 | 9, 12, 15, 18 | IP |
| 4 | Peptide SEQ ID NO: 1 + anti-PDL1 | Peptide SEQ ID NO: 1 (9, 10, 11, 12, 13) anti-PDL1 (9, 12, 15, 18) | IP |
| 5 | CX4945 + anti-PDL1 | CX4945 (9, 10, 11, 12, 13) anti-PDL1 (9, 12, 15, 18) | O + IP |

(continued)

| Groups | Test substance | Days of test substance administration | Route of administration |
|---|---|---|---|
| 6 | PBS | 9, 10, 11, 12, 13 | IP |
| **IP:** intraperitoneal immunization, **O:** twice daily oral administration. | | | |

As shown in Figure 25, treatment with the peptide identified as SEQ ID NO: 1 significantly reduced tumor progression. Furthermore, a more marked decrease in tumor volume was observed when the administration of said peptide was combined with an anti-PDL1 antibody. This effect was not observed when mice were treated with CX4945 alone or combined with anti-PDL1 therapy.

These results indicate that the peptide identified as SEQ ID NO: 1, by inducing PDL1 expression in tumor cells, increases the probability of blocking an anti-PDL1 therapy and thus enhances the induction of a cytotoxic immune response against the tumor.

**Claims**

1. Use of the peptide identified as SEQ ID NO: 1 for the manufacture of a medicament for the induction of antitumor and antiviral immunity mediated by immunogenic cell death.

2. The use of claim 1 wherein the induction of antitumor and antiviral immunity comprises the in vivo and in vitro activation and differentiation of dendritic cells.

3. The use of claim 1 wherein the induction of antitumor immunity comprises the enhancement of the cytotoxic activity of NK cells or the activity of cytotoxic T cells at the tumor site.

4. The use of claim 1 wherein the induction of antitumor immunity comprises the reversion of immunosuppressive to immunostimulatory tumor microenvironment.

5. The use of claim 1 wherein the induction of antitumor and antiviral immunity comprises increasing the secretion of pro-inflammatory cytokines.

6. The use of claim 1 wherein the induction of antiviral immunity comprises the enhancement of a specific humoral immune response against viral antigens.

7. Method of treatment of cancer or viral infections wherein a therapeutically effective amount of a medicament for the induction of antitumor and antiviral immunity mediated by immunogenic cell death is administered to a subject in need, wherein the medicament comprises the peptide identified as SEQ ID NO: 1.

8. The method of claim 7 wherein the induction of antitumor and antiviral immunity comprises the in vivo and in vitro activation and differentiation of dendritic cells.

9. The method of claim 7 wherein the induction of antitumor immunity comprises enhancing the cytotoxic activity of NK cells or the activity of cytotoxic T cells at the tumor site.

10. The method of claim 7 wherein the induction of antitumor immunity comprises reversion of immunosuppressive to immunostimulatory tumor microenvironment.

11. The method of claim 7 wherein the induction of antitumor and antiviral immunity comprises increasing the secretion of pro-inflammatory cytokines.

12. The method of claim 7 wherein the induction of antiviral immunity comprises enhancing of a specific humoral immune response against viral antigens.

13. The method of claim 7 wherein the medicament comprising the peptide identified as SEQ ID NO: 1 enhances the effect of a vaccine used in cancer immunotherapy.

**14.** The method of claim 13 wherein the medicament comprising the peptide identified as SEQ ID NO: 1 and the vaccine used in cancer immunotherapy are administered sequentially or simultaneously in the course of same treatment.

**15.** The method of claim 14 wherein the vaccine used in cancer immunotherapy is a vaccine based on dendritic cells, on cytotoxic T cells or on tumor-infiltrating lymphocytes.

**16.** Pharmaceutical combination comprising the peptide identified as SEQ ID NO: 1 and a vaccine for cancer immunotherapy.

**17.** The combination of claim 16 wherein the vaccine used in cancer immunotherapy is a vaccine based on dendritic cells, cytotoxic T cells or tumor-infiltrating lymphocytes.

**18.** The method of claim 7 wherein the medicament comprising the peptide identified as SEQ ID NO: 1 enhances the effect of a PDL1 immune checkpoint inhibitor used in cancer immunotherapy.

**19.** The method of claim 18 wherein the medicament comprising the peptide identified as SEQ ID NO: 1 and the PDL1 immune checkpoint inhibitor are administered sequentially or simultaneously in the course of same treatment.

**20.** The method of claim 18 wherein the PDL1 immune checkpoint inhibitor is an anti-PDL1 monoclonal antibody.

**21.** Pharmaceutical combination comprising the peptide identified as SEQ ID NO: 1 and a PDL1 immune checkpoint inhibitor used in cancer immunotherapy.

**22.** The pharmaceutical combination of claim 21 wherein the PDL1 immune checkpoint inhibitor is an anti-PDL1 monoclonal antibody.

**Figure 1**

**Figure 2**

**Figure 3**

**Figure 4**

**Figure 5**

## Figure 6

## Figure 7

## Figure 8

## Figure 9

## Figure 10

## Figure 11

**A**

**B**

**Figure 12**

**Figure 13**

**Figure 14**

**Figure 15**

Figure 16

Figure 17

## Figure 18

## Figure 19

## Figure 20

## Figure 21

## Figure 22

## Figure 23

**A**

**B**

## Figure 24

**A**

**B**

**Figure 25**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TARTARI F.** *Cancer Treat Rev,* 2016, vol. 48, 20-24
- **PEREA SE.** *Cancer Res,* 2004, vol. 64, 7129-9
- **PERERA Y.** *Mol Cancer Ther,* 2009, vol. 8 (5
- **FARINA HG.** *Exp Cell Res,* 2011, vol. 317, 1677-1688
- **BENAVENT ACERO F.** *Lung Cancer,* 2017, vol. 107, 14-21
- **MARTINS LR.** *Oncotarget,* 2014, vol. 5, 258-263
- **BATISTA-ALBUERNE N.** *J Med Oncol,* 2018, vol. 1, 4
- **SOLARES AM.** *BMC Cancer,* 2019, vol. 9 (1), 146
- **SARDUY MR.** *Br J Cancer,* 2015, vol. 112, 1636-43
- **ZUGAZAGOITIA J.** *Clin Ther,* 2016, vol. 38 (7), 1551-1566
- **YANG Y.** *J Clin Invest,* 2015, vol. 125 (9), 3335-3337
- **KENDERIAN SS.** *Biol Blood Transplant,* 2017, vol. 23, 235-246
- **RUELLA M ; KENDERIAN SS.** *BioDrug,* 2017, vol. 31 (6), 473-481
- **KLENER P JR.** *Curr Pharm Biotechnol,* 2015, vol. 16 (9), 771-781
- **LEE VC.** *P&T,* 2017, vol. 42 (6), 375-383
- **GARG AD.** *Oncoimmunology,* 2017, vol. 6 (12), e1386829
- **LI X.** *Tumori Jornal,* 2018, vol. 104 (1), 1-8
- **ROCK KL ; KONO H.** *Annu Rev Pathol,* 2008, vol. 3, 99-126
- **MATZINGER P.** *Science,* 2002, vol. 296, 301-305
- **HOU W.** *Cell Death and Disease,* 2013, vol. 4, e966
- **LIU X.** *Jornal of Hematology & Oncology,* 2017, vol. 10, 12
- **TONG B ; WANG M.** *Future Oncol.,* 2018, vol. 14 (21), 2179-2188
- **WEISKOPFK.** *Science,* 2013, vol. 341 (6141), 88-91
- **VANDENBERK L.** *Front Immunol,* 2016, vol. 6, 663
- **KEEP O.** *Oncoimmunology,* 2014, vol. 3, 9
- **MA Y.** *Immunity,* 2013, vol. 38, 729-41
- **ZITVOGEL L.** *Cell,* 2010, vol. 140, 798-804
- **MONTICO B.** *Int J Mol Sci.,* 2018, vol. 19, 594-609
- **CHEN HM.** *Cancer Immunol Immunother.,* 2012, vol. 61, 1989-2002
- **MONTICO B.** *Oncoimmunology,* 2017, vol. 6, e1356964
- **PEREA SE.** *Cáncer res.,* 2004, vol. 64, 7127-7129
- **VÁZQUEZ-BLOMQUIST D.** *Viral Immunol.,* 2002, vol. 5 (2), 337-356
- **GRANADILLO M. et al.** *Vaccine,* 2019, vol. 37 (30), 3957-3960